# EUROPEAN PATENT SPECIFICATION

(11) **EP 4 056 140 B1**
(45) Date of publication and mention of the grant of the patent: **04.11.2026**
(21) Application number: 20885752.4
(22) Date of filing: 05.11.2020
(51) Int. Cl.: A61B 34/20, G06N 20/00, G16H 40/20, G16H 40/63, A61B 34/00, A61B 34/32, A61B 17/00, G06F 3/01, G06F 3/16

(54) **INSTRUMENT-TO-BE-USED ESTIMATION DEVICE AND METHOD, AND SURGERY ASSISTANCE ROBOT**
VORRICHTUNG UND VERFAHREN ZUR KALKULATION EINES ZU BENUTZENDEN INSTRUMENTS SOWIE OPERATIONSUNTERSTÜTZUNGSROBOTER
DISPOSITIF ET PROCÉDÉ D'ESTIMATION D'INSTRUMENT À UTILISER, ET ROBOT D'ASSISTANCE CHIRURGICALE

(30) Priority: 07.11.2019 JP 2019202763
(43) Date of publication of application: 14.09.2022
(73) Proprietor: Kawasaki Jukogyo Kabushiki Kaisha, Kobe-shi, Hyogo 650-8670 (JP)
(72) Inventor: HASHIMOTO, Yasuhiko, Kobe-shi, Hyogo 650-8670 (JP)
(74) Representative: Müller-Boré & Partner Patentanwälte PartG mbB
(86) International application number: PCT/JP2020/041319
(87) International publication number: WO 2021/090870

(56) References cited:
- WO-A1-2015/145358
- WO-A1-2019/111512
- WO-A1-2019/111512
- JP-A- 2019 022 580
- US-A1- 2014 012 793
- US-A1- 2019 223 961
- US-A1- 2020 312 464
- TIAN ZHOU ET AL: "Spiking Neural Networks for Early Prediction in Human Robot Collaboration", ARXIV.ORG, CORNELL UNIVERSITY LIBRARY, 201 OLIN LIBRARY CORNELL UNIVERSITY ITHACA, NY 14853, 29 July 2018 (2018-07-29), XP081252111
- NISHIO SHOICHI ET AL: "Real-Time Orthopedic Surgery Procedure Recognition Method with Video Images from Smart Glasses Using Convolutional Neural Network", 2018 IEEE INTERNATIONAL CONFERENCE ON SYSTEMS, MAN, AND CYBERNETICS (SMC), IEEE, 7 October 2018 (2018-10-07), pages 379 - 384, XP033502654, DOI: 10.1109/SMC.2018.00074

## Description

### TECHNICAL FIELD

The present invention relates to a technique of predicting a surgical instrument suitable for a surgery situation during a surgical operation.

### BACKGROUND ART

At surgical operation sites, a surgical instrument to be used by a surgeon is handed to the surgeon from a surgical room nurse (instrument handling nurse). Before a demand is verbally told from the surgeon, the nurse under a surgical operation predicts a surgical instrument to be used next based on the self experiences in consideration of the progress state of the surgery and the surgeon's motion, and prepares so that he/she can promptly pass it to the surgeon. Such an instrument handling work of the nurse is based on tacit knowledge, such as the surgeon's habit, operating pattern, etc.

In recent years, the technology for automatically recognizing a human motion by using sensor data obtained from a sensor apparatus etc. attracts the attention. For example, Patent Document 1 discloses a dynamic state recognition device which acquires information from a dynamic state sensor (wearable sensor) attached to a subject person, such as a worker, and recognizes a dynamic state item indicative of each state of the contents of the dynamic state performed by the subject person. This dynamic state recognition device detects the dynamic state of the subject by using the information from the dynamic state sensor, narrows down the dynamic state item based on the detected dynamic state, and performs recognition processing for the narrowed-down dynamic state item. Further, Patent Document 2 discloses a recognition device which acquires, from a camera, image data in which person's motion is projected, and predicts a motion recognized result (context). This recognition device has a selection engine and a plurality of different recognition engines. The selection engine automatically selects one or more recognition engines optimal for input data, and the motion recognized result which is derived by the selected recognition engine is outputted.

### [Reference Documents of Conventional Art]

### [Patent Documents]

[Patent Document 1] JP2010-148604A
[Patent Document 2] JP2019-139479A

The following document introduces the Turn-Taking Spiking Neural Network (TTSNet), which is a cognitive model to perform early turn-taking prediction about human or agent's intentions. The TTSNet framework relies on implicit and explicit multimodal communication cues (physical, neurological and physiological) to be able to predict when the turn-taking event will occur in a robust and unambiguous fashion:
*TIAN ZHOU ET AL: "Spiking Neural Networks for Early Prediction in Human Robot Collaboration",*
ARXIV.ORG, CORNELL UNIVERSITY LIBRARY, 201 OLIN LIBRARY CORNELL UNIVERSITY ITHACA, NY 14853, 29 July 2018*.*
WO 2019/111512 A1 describes a medical information processing device which comprises: a selection unit that selects, from candidate data which includes surgery data obtained during a surgery, data of interest that corresponds to selection criteria including a criterion relating to patient attributes, a criterion relating to the procedure type, and a criterion relating to the disease type; an extraction unit that detects characteristics from the selected data of interest and extracts, from the data of interest, characteristics data that corresponds to the detected characteristics; and an editing unit that edits the extracted characteristics data. The extraction unit extracts at least a medical image as the characteristics data.
US 2019/223961 A1 describes that a system for robot-assisted surgery includes an image sensor and a display. The system further includes a controller coupled to the image sensor and the display.

The following document proposes a computer-aided navigation system that indicates the current procedure and procedure progress for nurses, and also instructs nurses to prepare surgical instruments to be used in the next procedure using smart glasses:
*NISHIO SHOICHI ET AL: "Real-Time Orthopedic Surgery Procedure Recognition Method with Video Images from Smart Glasses Using Convolutional Neural Network",*
2018 IEEE INTERNATIONAL CONFERENCE ON SYSTEMS, MAN, AND CYBERNETICS (SMC), IEEE, 7 October 2018 (2018-10-07), pages 379-384*,*
*DOI: 10.1109*/*SMC.2018.00074*
US 2014/012793 A1 describes that system and method for predicting a surgery progress stage sense a surgery motion pattern according to a surgery motion, store surgery stage information according to the kind of surgery and pattern information of major surgery motions of each surgery stage, determine a present surgery motion pattern corresponding to the sensed surgery motion pattern from the surgery motion pattern information stored in the data storing unit, and predict a surgery stage corresponding to the determined present surgery motion pattern based on the surgery stage information stored in the data storing unit.

### DESCRIPTION OF THE INVENTION

### [Problem(s) to be Solved]

Good or bad of the instrument handling work of the surgical room nurse as described above largely depends on the nurse's skill and skill level. Further, since the instrument handling nurse stays in a tensed state for a long period of time, or stays in a standing state, he/she becomes greatly fatigued physically as well as mentally.

### [Means for Solving the Problem(s)]

The present inventors found out to perform by a computer at least a part of an instrument handling work, which is conventionally conducted by a surgical room nurse, that is, a selection of a surgical instrument suitable for a surgery situation. By this technique, the surgical instrument suitable for the surgery situation can be provided to the surgeon without depending on the nurse's skill and skill level. Further, it makes possible for a robot to pass the surgical instrument to the surgeon, which contributes to the reduction of fatigues of the surgical room nurse.

A use instrument predicting device according to the present invention is defined in claim 1.

Further, a use instrument prediction method according to the present invention is defined in claim 3.

A robot control device may operate a manipulator arm so that the manipulator arm transfers the surgical instrument to a given handing position. The given handing position may be a fixed position defined according to the contents of the surgical operation, a position defined beforehand according to the progress state of the surgical operation, or the surgeon's favorite handing position.

For example, the motion detection data may be at least one of data detected by a dynamic state sensor attached to the surgeon and imaging data obtained by a camera which images the surgeon's motion.

The situation detection data may include the contents of the surgical operation, and a lapsed time of the surgical operation. Note that the surgery situation may include a progress of the surgical operation (surgical processes, such as incision, excision, and suture).

Note that the kinds of surgical instruments which are generally used for surgical operations are roughly divided into a needle holder, tweezers, a needle and thread, forceps, a hook, a retractor, etc. These major classifications of the surgical instruments are further finely classified depending on the shape of the tip end, the size and the use.

### [Effect of the Invention]

According to the present invention, prediction of a surgical instrument suitable for a surgery situation, which is conventionally conducted by a surgical room nurse, can be performed automatically without depending on the nurse's skill and skill level.

### BRIEF DESCRIPTION OF DRAWINGS

Fig. 1 is a view illustrating the entire configuration of a surgery assisting robot according to one embodiment of the present disclosure.
Fig. 2 is a schematic configuration diagram of a robot control device.
Fig. 3 is a schematic configuration diagram of a use instrument predicting device.
Fig. 4 is a functional block diagram of the use instrument predicting device.
Fig. 5 is a view illustrating a flow of processing of a motion recognizing module.
Fig. 6 is a view illustrating a flow of processing of a situation recognizing module.
Fig. 7 is a view illustrating a flow of processing of a predicting module.
Fig. 8 is a flowchart of processing of the use instrument predicting device.
Fig. 9 is a flowchart of the processing of the use instrument predicting device.

### MODES FOR CARRYING OUT THE DISCLOSURE

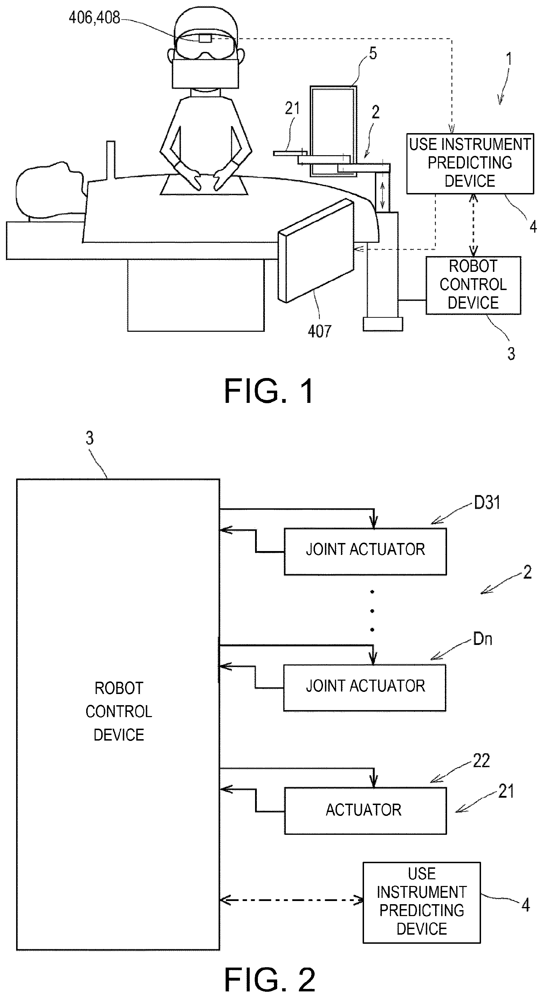

Next, one embodiment of the present disclosure is described with reference to the drawings. Fig. 1 is a view illustrating the entire configuration of a surgery assisting robot 1 according to one embodiment of the present invention. The surgery assisting robot 1 illustrated in Fig. 1 includes at least one manipulator arm 2, a robot control device 3, a use instrument predicting device 4, and an instrument storage 5. The instrument storage 5 stores a plurality of kinds of surgical instruments, which may be used for a surgery, in a sterile condition.

### [Manipulator Arm 2]

The manipulator arm 2 is an articulated robotic arm having a plurality of joints. A hand 21 is attached to a distal-end part of the manipulator arm 2 as an end effector. The hand 21 is capable of grasping a surgical instrument. Although in this embodiment a horizontal articulated robotic arm is adopted as the manipulator arm 2, any kind of robotic arm can be adopted as long as the manipulator arm 2 is an articulated robotic arm. Further, the manipulator arm 2 may be installed by a surgical table, or may be suspended from the ceiling of a surgical room.

### [Robot Control Device 3]

The robot control device 3 is provided with a computer, and by a processor performing a program, or a decryption and a calculation of various signals inputted from the use instrument predicting device 4, it controls the motion of the manipulator arm 2 and the motion of the hand 21, and outputs signals from various output ports.

As illustrated in Fig. 2, the manipulator arm 2 is provided with joint actuators D31-Dn corresponding to the respective joints. For example, each of the joint actuators D31-Dn is comprised of a servomotor, a power transmission mechanism which transmits the power of the servomotor to the joint, a reduction gear which adjusts output torque of the servomotor and transmits it to the power transmission mechanism, and a rotational position sensor which detects a rotational position of the servomotor (none of them is illustrated). The robot control device 3 controls the position of a control point of the manipulator arm 2 (for example, a point on the hand 21) by acquiring a detection signal of the rotational position sensor for each of the joint actuators D31-Dn, and adjusting the output of the servomotor based on the detection signal. The hand 21 includes at least one actuator 22. The robot control device 3 controls the operation of the hand 21 by operating the actuator 22.

### [Use Instrument Predicting Device 4]

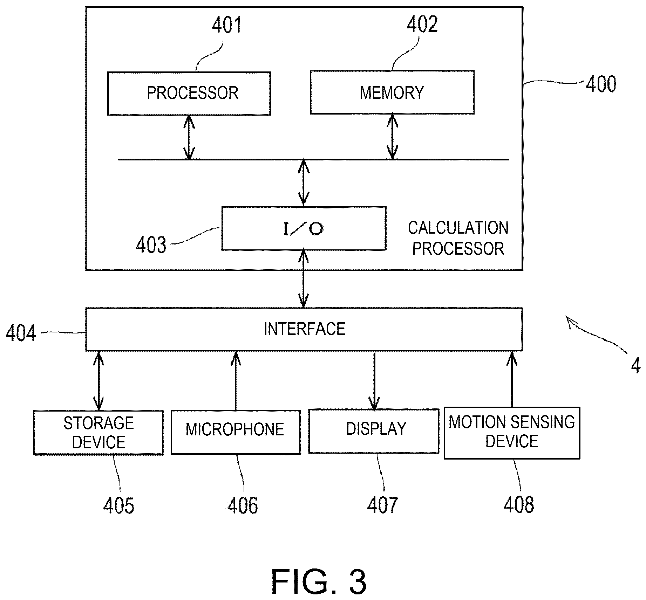

The use instrument predicting device 4 is provided with a calculation controller 400. Each functional part (described later) of the use instrument predicting device 4 may be comprised of at least one calculation controller 400, or two or more of a plurality of functional parts may be comprised of one calculation controller 400. As illustrated in Fig. 3, each calculation controller 400 of the use instrument predicting device 4 includes a processor 401, a memory 402 such as a ROM and a RAM, and an I/O part 403. The calculation controller 400 is connected with a storage device 405, a microphone 406, a display 407, and a motion sensing device 408 via an interface 404. The motion sensing device 408 may be at least one of a plurality of dynamic state sensors attached to a surgeon, and a camera which images the surgeon's motion and an affected part.

The calculation controller 400 may be provided with a sole processor 401 which performs a centralized control, or may be provided with a plurality of processors 401 which perform a distributed control. For example, the calculation controller 400 may be comprised of at least one of, or a combination of two or more of a computer, a personal computer, a microcontroller, a microprocessor, a PLD (Programmable Logic Device) such as a FPGA (Field-Programmable Gate Array), a PLC (Programmable Logic Controller), and a logical circuit. The memory 402 or the storage device 405 stores a basic program, a software program, etc. which are executed by the processor 401. By the processor 401 reading and executing the program, the calculation controller 400 realizes a function configured in the software program.

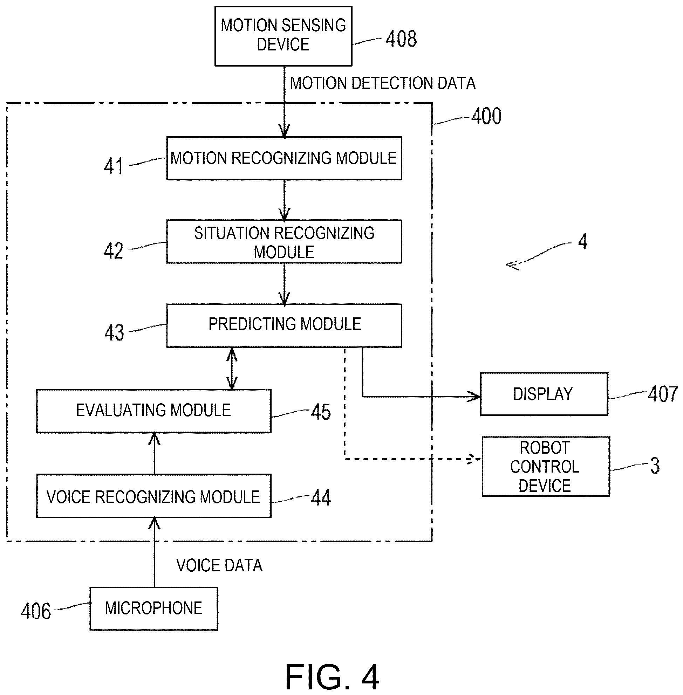

As illustrated in Fig. 4, the use instrument predicting device 4 includes functional parts, such as a motion recognizing module 41, a situation recognizing module 42, a predicting module 43, a voice recognizing module 44, and an evaluating module 45. The use instrument predicting device 4 is also provided with a learning module (not illustrated) corresponding to each of the motion recognizing module 41, the situation recognizing module 42, and the predicting module 43.

### [Motion Recognizing Module 41]

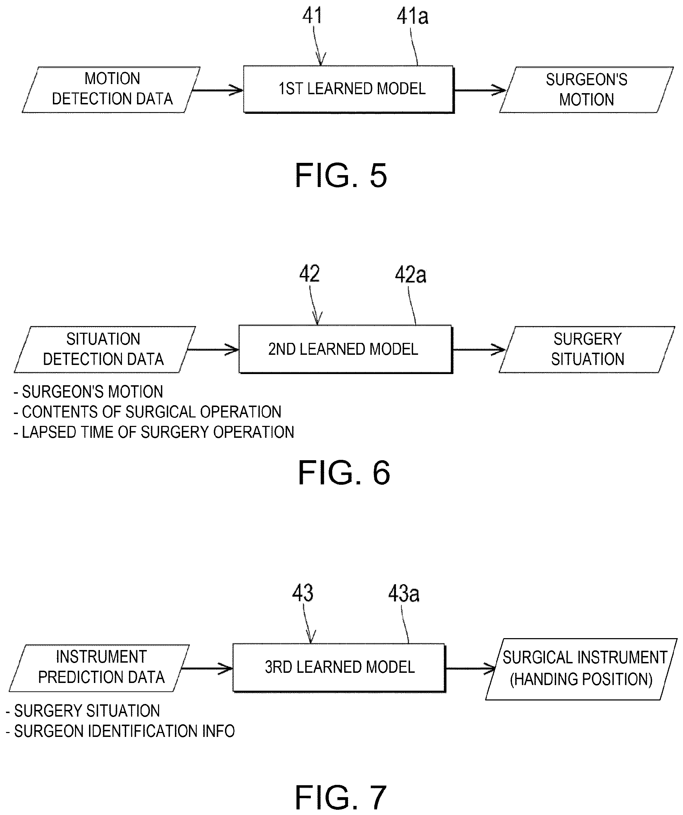

As illustrated in Fig. 5, the motion recognizing module 41 recognizes a surgeon's motion based on motion detection data acquired from the motion sensing device 408. For example, the motion detection data may be at least one of data detected by the dynamic state sensor attached to the surgeon and imaging data obtained by the camera which images the surgeon's motion. Further, the motion detection data may include surgeon identification information for identifying a surgeon. The motion recognizing module 41 has a first learned model 41a which has learned with a large number of first teaching data in which the motion detection data and the surgeon's motion which are accumulated are associated with each other. The motion recognizing module 41 derives the surgeon's motion to be recognized corresponding to the motion detection data by using the first learned model 41a. For example, the motion recognizing module 41 can calculate a moving amount of the surgeon's specific part based on the motion detection data, and can recognize the surgeon's motion based on the moving amount. Note that the motion recognition technology has already been a known technology, and the motion recognizing module 41 can utilize the known motion recognition technology.

The learning module of the motion recognizing module 41 creates the first learned model 41a by using the machine learning technology. The learning module of the motion recognizing module 41 preprocesses the motion detection data to create the first teaching data. The preprocess includes at least one of various processings, such as a conversion of data format, a check of abnormality, an extraction of data, and a change of variable identifier or filename. The learning module of the motion recognizing module 41 learns a correlation between input data and output data by the machine learning. In this embodiment, the input data is the motion detection data and the output data is the surgeon's motion. The learning module of the motion recognizing module 41 performs supervised learning (class classification) as one example of the machine learning. Generally, the supervised learning is a technique in which a large number of known data sets (teaching data) comprised of input data and corresponding output data are given beforehand, and a feature which suggests a correlation between the input data and the output data is identified based on the teaching data using a learning algorithm to learn a correlation model for predicting necessary output data for new input data. This correlation model becomes the first learned model 41a. The first learned model 41a is stored in the storage device 405. The first teaching data in an early stage of learning is an accumulation of data set which includes the surgeon's motion detected by the motion sensing device 408 during an actual surgical operation, and the surgeon's motion which is given as a correct answer.

### [Situation Recognizing Module 42]

As illustrated in Fig. 6, the situation recognizing module 42 recognizes a surgery situation based on situation detection data which includes the recognized result of the motion recognizing module 41 (i.e., the surgeon's motion). The situation detection data may include the contents of the surgical operation, and a lapsed time of the surgical operation. The situation recognizing module 42 has a second learned model 42a which has learned with a large number of second teaching data in which the situation detection data and the surgery situation which are accumulated are associated with each other. The situation recognizing module 42 derives the surgery situation to be recognized corresponding to the situation detection data by using the second learned model 42a. Note that the surgery situation may include a progress of the surgical operation (surgical processes, such as incision, excision, and suture).

The learning module of the situation recognizing module 42 creates the second learned model 42a by using the machine learning technology. The learning method of the learning module of the situation recognizing module 42 may be similar to that of the learning module of the motion recognizing module 41. The second teaching data in an early stage of learning is an accumulation of data set which includes the situation detection data obtained during an actual surgical operation, and the surgery situation which is given as a correct answer.

### [Predicting Module 43]

As illustrated in Fig. 7, the predicting module 43 predicts at least one kind of surgical instrument to be used next by the surgeon based on instrument prediction data including the situation recognized result of the situation recognizing module 42 (i.e., the surgery situation). The instrument prediction data includes the surgeon identification information for identifying the surgeon. Based on the surgeon identification information, the surgeon's dominant hand, his/her favorite surgical instrument, and his/her favorite handing position can be identified. The predicting module 43 has a third learned model 43a which has learned with a large number of third teaching data in which the instrument prediction data and the kind of surgical instrument which are accumulated are associated with each other. The predicting module 43 derives a kind of the surgical instrument corresponding to the instrument prediction data to be recognized using the third learned model 43a. Note that the kinds of surgical instruments which are generally used for surgical operations are roughly divided into a needle holder, tweezers, a needle and thread, forceps, a hook, the retractor, etc. These major classifications of the surgical instruments are further finely classified depending on the shape of the tip end, the size and the use.

The third learned model 43a may derive the handing position according to the kind of surgical instrument. The handing position may be a fixed position defined according to the contents of the surgical operation, a position defined beforehand according to the progress state of the surgical operation, or the surgeon's favorite handing position. Alternatively, the handing position may be identified by absolute coordinates, or may be identified as a position which opposes to the surgical table or the manipulator arm 2.

The learning module of the predicting module 43 creates the third learned model 43a using the machine learning technology. The learning method of the learning module of the predicting module 43 may be similar to that of the learning module of the motion recognizing module 41. The third teaching data in an early stage of learning is an accumulation of data set which includes the instrument prediction data obtained during an actual surgical operation, and the kind of surgical instrument which is given as a correct answer.

### [Voice Recognizing Module 44]

The microphone 406 acquires a voice analog signal (i.e., voice), and converts it into a voice digital signal. The voice recognizing module 44 acquires the voice digital signal, and converts it into text data. For example, the voice recognizing module 44 converts the inputted voice into a voice wave, conducts an acoustic analysis of the voice wave to identify the phoneme, performs a matching of the row of the phoneme with a dictionary registered beforehand to convert it into a word, and outputs the converted sentence as text data. The speech recognition technology using machine learning is widely known, and the voice recognizing module 44 is constituted utilizing a known speech recognition technology.

### [Evaluating Module 45]

The evaluating module 45 determines whether the prediction result by the predicting module 43 is correct. Processing of the evaluating module 45 will be described later.

### [Surgical Operation Assisting Method]

Here, a surgical operation assisting method using the surgery assisting robot 1 is described. This surgical operation assisting method utilizes the use instrument prediction method according to this embodiment.

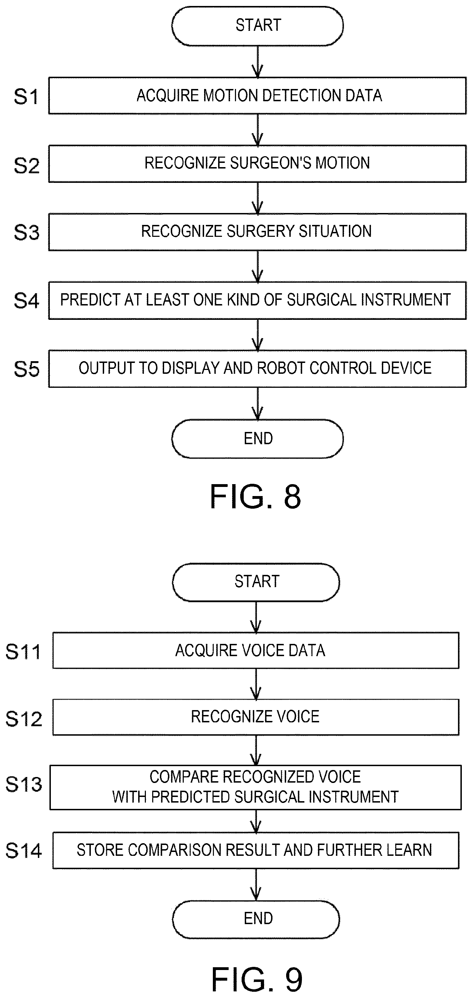

Figs. 8 and 9 are flowcharts of processing of the use instrument predicting device 4. As illustrated in Fig. 8, the use instrument predicting device 4 acquires the motion detection data which is obtained by detecting the surgeon's motion under a surgical operation from the motion sensing device 408 (Step S1).

Next, the motion recognizing module 41 of the use instrument predicting device 4 recognizes the surgeon's motion based on the motion detection data (Step S2). Then, the situation recognizing module 42 of the use instrument predicting device 4 recognizes the surgery situation based on the motion recognized result (i.e., the surgeon's motion) of the motion recognizing module 41 (Step S3). Further, the predicting module 43 of the use instrument predicting device 4 predicts at least one kind of surgical instrument to be used next by the surgeon out of a plurality of kinds of surgical instruments given beforehand, based on the situation recognized result (i.e., the surgery situation) of the situation recognizing module 42 (Step S4).

The use instrument predicting device 4 outputs the prediction result of the surgical instrument to the display 407 (Step S5). The display 407 is disposed at a position where the surgeon inside the surgical room can view. The surgeon can visually recognize the information on the surgical instrument which will be provided next, which is displayed on and outputted to the display 407.

Further, the use instrument predicting device 4 outputs the prediction result of the surgical instrument to the robot control device 3 (Step S5). The robot control device 3, which received the prediction result of the surgical instrument, operates the manipulator arm 2 so that it picks out at least one kind of predicted surgical instrument which will be used next by the surgeon from the instrument storage 5, and then stands by at a standby position.

The surgeon utters voice in order to demand a surgical instrument to be used next. For example, the surgeon utters as "scalpel." This voice is collected by the microphone 406 and transmitted to the use instrument predicting device 4.

As illustrated in Fig. 9, the voice recognizing module 44 of the use instrument predicting device 4 acquires the voice data from the microphone 406 (Step S11), and performs the speech recognition (Step S12). The voice data is converted into the voice text data by the speech recognition.

The evaluating module 45 of the use instrument predicting device 4 compares the voice text data (i.e., the voice recognized) with the information on the surgical instrument predicted by the predicting module 43, and determines a match or a non-match (Step S13). The use instrument predicting device 4 stores the determination result, creates new teaching data including the motion detection data, the surgical instrument predicted based on the motion detection data, and the determination result, and causes the learned models 41a, 42a, and 43a to further learn (Step S14).

If determined matched at Step S13, the use instrument predicting device 4 outputs an enabling signal to the robot control device 3. The robot control device 3 which received the enabling signal operates the manipulator arm 2 so that the manipulator arm 2 moves from the standby position to the handing position. Therefore, the surgical instrument demanded by the surgeon can be provided promptly and correctly.

On the other hand, if determined non-matched at Step S13, the use instrument predicting device 4 outputs the prediction result of at least one surgical instrument which is derived from the voice text data, and an enabling signal to the robot control device 3. The robot control device 3 which received the prediction result of the surgical instrument operates the manipulator arm 2 so that the manipulator arm 2 picks out at least one kind of predicted surgical instrument to be used next by the surgeon from the instrument storage 5, and moves it to the handing position. Therefore, the surgical instrument demanded by the surgeon can be provided correctly.

As described above, the use instrument predicting device 4 according to this embodiment includes the motion recognizing module 41 which recognizes the surgeon's motion during a surgical operation based on the motion detection data which is obtained by detecting the surgeon's motion, the situation recognizing module 42 which recognizes the surgery situation based on the motion recognized result of the motion recognizing module 41, and the predicting module 43 which predicts at least one kind of surgical instrument to be used next by the surgeon out of the plurality of kinds of surgical instruments given beforehand, based on the situation recognized result of the situation recognizing module 42.

Moreover, the use instrument prediction method according to this embodiment includes the steps of acquiring the motion detection data which is obtained by detecting the surgeon's motion during a surgical operation, recognizing the surgeon's motion based on the motion detection data, recognizing the surgery situation based on the recognized surgeon's motion, and predicting at least one kind of surgical instrument to be used next by the surgeon out of the plurality of kinds of surgical instruments given beforehand, based on the recognized surgery situation.

The surgery assisting robot 1 according to this embodiment includes the use instrument predicting device 4, the at least one manipulator arm 2, the robot control device 3 which controls the operation of the manipulator arm 2, and the instrument storage 5 which accommodates the plurality of kinds of surgical instruments. The robot control device 3 operates the manipulator arm 2 so that the manipulator arm 2 picks out from the instrument storage 5 the at least one kind of surgical instrument to be used next by the surgeon, which is predicted by the use instrument predicting device 4, and transfers it to the given handing position.

According to the use instrument predicting device 4, the method, and the surgery assisting robot 1, which are described above, the prediction of the surgical instrument suitable for the surgery situation, which is conventionally conducted by the surgical room nurse, can be performed automatically without depending on the nurse's skill and skill level.

In the use instrument predicting device 4, the motion recognizing module 41 includes the first learned model 41a learned with the large number of first teaching data in which the motion detection data is associated with the surgeon's motion, and derives the surgeon's motion to be recognized corresponding to the motion detection data using the first learned model 41a.

In the use instrument predicting device 4, the situation recognizing module 42 includes the second learned model 42a learned with the large number of second teaching data in which the situation detection data including the motion recognized result is associated with the surgery situation, and derives the surgery situation to be recognized corresponding to the situation detection data using the second learned model 42a.

In the use instrument predicting device 4, the predicting module 43 includes the third learned model 43a learned with the large number of third teaching data in which the instrument prediction data including the situation recognized result is associated with the kind of surgical instrument, and derives the kind of surgical instrument to be recognized corresponding to the instrument prediction data using the third learned model 43a.

Further, in the use instrument predicting device 4, the predicting module 43 derives the handing position which is the position where the predicted surgical instrument is passed to the surgeon.

According to the use instrument predicting device 4 having the above configuration, the surgical instrument to be demanded next by the surgeon can be estimated correctly.

In the above embodiment, when at least one kind of surgical instrument which is predicted by the use instrument predicting device 4 differs from the surgical instrument demanded by the surgeon, the manipulator arm 2 goes to the instrument storage 5 to again take out the demanded surgical instrument, and carries it to the handing position. However, the manipulator arm 2 may return the surgical instrument to the instrument storage 5 while the demanded surgical instrument may be handed to the surgeon from a nurse. Further, when the handing position differs from the position demanded by the surgeon, the nurse may move the manipulator arm 2 to the correct handing position, the robot control device 3 may then acquire the correct handing position, and the use instrument predicting device 4 may learn the position. In order for the nurse to be able to move the manipulator arm 2, the manipulator arm 2 may be provided with a hand guide button. The manipulator arm 2 serves as a hand guide robot while the hand guide button is pushed, and the robot control device 3 controls the operation of the manipulator arm 2 so that the manipulator arm 2 is freely movable by an external force given by the nurse.

Although in the above embodiment the learned models 41a, 42a, and 43a provided to the use instrument predicting device 4 learn every time the series of prediction processings is finished, the additional learning may be performed after a plurality of prediction processings are finished and a certain amount of teaching data is accumulated.

### DESCRIPTION OF REFERENCE CHARACTERS

- 1:: Surgery Assisting Robot
- 2:: Manipulator Arm
- 3:: Robot Control Device
- 4:: Use Instrument Predicting Device
- 5:: Instrument Storage
- 400:: Calculation Controller
- 41:: Motion Recognizing Module
- 41a, 42a, 43a, 49a:: Learned Model
- 42:: Situation Recognizing Module
- 43:: Predicting Module
- 49:: Calculation Module

## Claims

1. A use instrument predicting device (4), comprising:
a motion recognizing module (41) that recognizes a motion of a surgeon during a surgical operation based on motion detection data that is obtained by detecting the surgeon's motion;
a situation recognizing module (42) that recognizes a surgery situation based on the motion recognized result of the motion recognizing module (41); and
a predicting module (43) that predicts at least one kind of surgical instrument to be used next by the surgeon out of a plurality of kinds of surgical instruments given beforehand, based on the situation recognized result of the situation recognizing module (42),
wherein the motion recognizing module (41) has a first learned model (41a) learned with a large number of first teaching data in which the motion detection data is associated with the surgeon's motion, and derives the surgeon's motion to be recognized corresponding to the motion detection data, by using the first learned model (41a),
wherein the situation recognizing module (42) has a second learned model (42a) learned with a large number of second teaching data in which situation detection data including the motion recognized result is associated with the surgery situation, and derives the surgery situation to be recognized corresponding to the situation detection data, by using the second learned model (42a),
wherein the predicting module (43) has a third learned model (43a) learned with a large number of third teaching data in which instrument prediction data including the situation recognized result is associated with the kind of surgical instrument, and derives the kind of surgical instrument to be recognized corresponding to the instrument prediction data, by using the third learned model (43a),
wherein the predicting module (43) derives a handing position that is a position where the predicted surgical instrument is passed to the surgeon, and
wherein the instrument prediction data further includes surgeon identification information for identifying the surgeon's dominant hand and the surgeon's favorite handing position.

2. A surgery assisting robot (1), comprising:
the use instrument predicting device (4) of claim 1;
at least one manipulator arm (2);
a robot control device (3) that controls operation of the manipulator arm (2); and
an instrument storage (5) that accommodates a plurality of kinds of surgical instruments,
wherein the robot control device (3) operates the manipulator arm (2) so that the manipulator arm (2) picks out the predicted surgical instrument from the instrument storage (5), and transfers the predicted surgical instrument to the handing position.

3. A use instrument prediction method, comprising the steps of:
acquiring motion detection data that is obtained by detecting a motion of a surgeon during a surgical operation;
recognizing the surgeon's motion based on the motion detection data, wherein the surgeon's motion to be recognized corresponding to the motion detection data is derived by using a first learned model (41a) learned with a large number of first teaching data in which the motion detection data is associated with the surgeon's motion;
recognizing a surgery situation based on the recognized surgeon's motion, wherein the surgery situation to be recognized corresponding to situation detection data including the motion recognized result is derived by using a second learned model (42a) learned with a large number of second teaching data in which the situation detection data is associated with the surgery situation;
predicting at least one kind of surgical instrument to be used next by the surgeon out of a plurality of kinds of surgical instruments given beforehand, based on the recognized surgery situation, wherein the kind of surgical instrument corresponding to instrument prediction data including the situation recognized result is derived by using a third learned model (43a) learned with a large number of third teaching data in which the instrument prediction data is associated with the kind of surgical instrument; and
deriving a handing position that is a position where the predicted surgical instrument is passed to the surgeon,
wherein the instrument prediction data further includes surgeon identification information for identifying the surgeon's dominant hand and the surgeon's favorite handing position.

## Patentansprüche

1. Verwendungsinstrument-Vorhersagevorrichtung (4), umfassend:
ein Bewegungserkennungsmodul (41), das eine Bewegung eines Chirurgen während einer chirurgischen Operation basierend auf Bewegungserfassungsdaten erkennt, die durch Erfassen der Bewegung des Chirurgen erhalten werden;
ein Situationserkennungsmodul (42), das eine Operationssituation basierend auf dem Bewegungserkennungsergebnis des Bewegungserkennungsmoduls (41) erkennt; und
ein Vorhersagemodul (43), das basierend auf dem Situationserkennungsergebnis des Situationserkennungsmoduls (42) zumindest eine Art von chirurgischem Instrument, das als Nächstes von dem Chirurgen verwendet werden soll, aus einer Mehrzahl von vorab festgelegten Arten von chirurgischen Instrumenten vorhersagt,
wobei das Bewegungserkennungsmodul (41) ein erstes gelerntes Modell (41a) aufweist, das mit einer großen Anzahl erster Trainingsdaten angelernt wurde, in denen die Bewegungserfassungsdaten mit der Bewegung des Chirurgen verknüpft sind, und die zu erkennende Bewegung des Chirurgen entsprechend den Bewegungserfassungsdaten unter Verwendung des ersten gelernten Modells (41a) ableitet,
wobei das Situationserkennungsmodul (42) ein zweites gelerntes Modell (42a) aufweist, das mit einer großen Anzahl zweiter Trainingsdaten angelernt wurde, in denen Situationserfassungsdaten, die das Bewegungserkennungsergebnis beinhalten, mit der Operationssituation verknüpft sind, und die zu erkennende Operationssituation entsprechend den Situationserfassungsdaten unter Verwendung des zweiten gelernten Modells (42a) ableitet,
wobei das Vorhersagemodul (43) ein drittes gelerntes Modell (43a) aufweist, das mit einer großen Anzahl dritter Trainingsdaten angelernt wurde, in denen Instrumentenvorhersagedaten, die das Situationserkennungsergebnis beinhalten, mit der Art von chirurgischem Instrument verknüpft sind, und die zu erkennende Art von chirurgischem Instrument entsprechend den Instrumentenvorhersagedaten unter Verwendung des dritten gelernten Modells (43a) ableitet,
wobei das Vorhersagemodul (43) eine Übergabeposition ableitet, die eine Position ist, an der das vorhergesagte chirurgische Instrument dem Chirurgen gereicht wird, und
wobei die Instrumentenvorhersagedaten ferner Chirurgenidentifikationsinformationen zum Identifizieren der dominanten Hand des Chirurgen und der bevorzugten Übergabeposition des Chirurgen beinhalten.

2. Operationsassistenzroboter (1), umfassend:
die Verwendungsinstrument-Vorhersagevorrichtung (4) nach Anspruch 1;
zumindest einen Manipulatorarm (2);
eine Robotersteuerungs- bzw. -regelungsvorrichtung (3), die einen Betrieb des Manipulatorarms (2) steuert bzw. regelt; und
eine Instrumentenaufbewahrung bzw. -ablage (5), die eine Mehrzahl von Arten chirurgischer Instrumente aufnimmt,
wobei die Robotersteuerungs- bzw. -regelungsvorrichtung (3) den Manipulatorarm (2) so betätigt, dass der Manipulatorarm (2) das vorhergesagte chirurgische Instrument von der Instrumentenablage (5) nimmt und das vorhergesagte chirurgische Instrument an die Übergabeposition überführt.

3. Verwendungsinstrument-Vorhersageverfahren, umfassend die Schritte:
Gewinnen von Bewegungserfassungsdaten, die durch Erfassen einer Bewegung eines Chirurgen während eines chirurgischen Eingriffs erhalten werden;
Erkennen der Bewegung des Chirurgen basierend auf den Bewegungserfassungsdaten, wobei die zu erkennende Bewegung des Chirurgen, die den Bewegungserfassungsdaten entspricht, unter Verwendung eines ersten gelernten Modells (41a) abgeleitet wird, das mit einer großen Anzahl erster Trainingsdaten angelernt wurde, in denen die Bewegungserfassungsdaten mit der Bewegung des Chirurgen verknüpft sind;
Erkennen einer Operationssituation basierend auf der erkannten Bewegung des Chirurgen, wobei die zu erkennende Operationssituation, die Situationserfassungsdaten einschließlich des Bewegungserkennungsergebnisses entspricht, unter Verwendung eines zweiten gelernten Modells (42a) abgeleitet wird, das mit einer großen Anzahl zweiter Trainingsdaten angelernt wurde, in denen die Situationserfassungsdaten mit der Operationssituation verknüpft sind;
Vorhersagen zumindest einer Art von chirurgischem Instrument, das als Nächstes von dem Chirurgen verwendet werden soll, aus einer Mehrzahl von vorab festgelegten Arten von chirurgischen Instrumenten basierend auf der erkannten Operationssituation, wobei die Art von chirurgischem Instrument, die Instrumentenvorhersagedaten einschließlich des Situationserkennungsergebnisses entspricht, unter Verwendung eines dritten gelernten Modells (43a) abgeleitet wird, das mit einer großen Anzahl dritter Trainingsdaten angelernt wurde, in denen die Instrumentenvorhersagedaten mit der Art von chirurgischem Instrument verknüpft sind; und
Ableiten einer Übergabeposition, die eine Position ist, an der das vorhergesagte chirurgische Instrument dem Chirurgen gereicht wird,
wobei die Instrumentenvorhersagedaten ferner Chirurgenidentifikationsinformationen zum Identifizieren der dominanten Hand des Chirurgen und der bevorzugten Übergabeposition des Chirurgen beinhalten.

## Revendications

1. Dispositif de prédiction d'instrument d'utilisation (4), comprenant :
un module de reconnaissance de mouvement (41) qui reconnaît un mouvement d'un chirurgien pendant une opération chirurgicale sur la base de données de détection de mouvement obtenues en détectant le mouvement du chirurgien ;
un module de reconnaissance de situation (42) qui reconnaît une situation chirurgicale sur la base du résultat de reconnaissance de mouvement du module de reconnaissance de mouvement (41) ; et
un module de prédiction (43) qui prédit au moins un type d'instrument chirurgical à utiliser ensuite par le chirurgien parmi une pluralité de types d'instruments chirurgicaux donnés au préalable, sur la base du résultat de situation reconnu par le module de reconnaissance de situation (42),
dans lequel le module de reconnaissance de mouvement (41) présente un premier modèle appris (41a) appris avec un grand nombre de premières données d'apprentissage dans lesquelles les données de détection de mouvement sont associées au mouvement du chirurgien, et déduit le mouvement du chirurgien à reconnaître correspondant aux données de détection de mouvement, en utilisant le premier modèle appris (41a),
dans lequel le module de reconnaissance de situation (42) présente un deuxième modèle appris (42a) appris avec un grand nombre de secondes données d'apprentissage dans lesquelles des données de détection de situation comportant le résultat de mouvement reconnu sont associées à la situation chirurgicale, et déduit la situation chirurgicale à reconnaître correspondant aux données de détection de situation, en utilisant le deuxième modèle appris (42a),
dans lequel le module de prédiction (43) présente un troisième modèle appris (43A) appris avec un grand nombre de troisièmes données d'apprentissage dans lesquelles des données de prédiction d'instrument comportant le résultat de situation reconnu sont associées au type d'instrument chirurgical, et déduit le type d'instrument chirurgical à reconnaître correspondant aux données de prédiction d'instrument, en utilisant le troisième modèle appris (43A),
dans lequel le module de prédiction (43) déduit une position de prise en main qui est une position dans laquelle l'instrument chirurgical prédit est remis au chirurgien, et
dans lequel les données de prédiction d'instrument comportent en outre des informations d'identification du chirurgien permettant d'identifier la main dominante du chirurgien et la position de prise en main préférée du chirurgien.

2. Robot d'assistance chirurgicale (1), comprenant :
le dispositif de prédiction d'instrument d'utilisation (4) selon la revendication 1 ;
au moins un bras manipulateur (2) ;
un dispositif de commande de robot (3) qui commande le fonctionnement du bras manipulateur (2) ; et
un stockage d'instruments (5) qui peut loger une pluralité de types d'instruments chirurgicaux,
dans lequel le dispositif de commande de robot (3) fait fonctionner le bras manipulateur (2) de sorte que le bras manipulateur (2) choisit l'instrument chirurgical prévu dans le stockage d'instruments (5), et transfère l'instrument chirurgical prévu à la position de prise en main.

3. Procédé de prédiction d'instrument d'utilisation, comprenant les étapes consistant à :
l'acquisition de données de détection de mouvement obtenues en détectant un mouvement d'un chirurgien pendant une opération chirurgicale ;
la reconnaissance du mouvement du chirurgien sur la base des données de détection de mouvement, dans lequel le mouvement du chirurgien à reconnaître correspondant aux données de détection de mouvement est déduit en utilisant un premier modèle appris (41a) appris avec un grand nombre de premières données d'apprentissage dans lequel la détection de mouvement des données sont associées au mouvement du chirurgien ;
la reconnaissance d'une situation chirurgicale sur la base du mouvement du chirurgien reconnu, dans lequel la situation chirurgicale à reconnaître correspondant à des données de détection de situation comportant le résultat reconnu de mouvement est déduite en utilisant un deuxième modèle appris (42a) appris avec un grand nombre de deuxièmes données d'enseignement dans lequel la situation des données de détection sont associées à la situation chirurgicale ;
la prédiction d'au moins un type d'instrument chirurgical à utiliser ensuite par le chirurgien parmi une pluralité de types d'instruments chirurgicaux donnés au préalable, sur la base de la situation chirurgicale reconnue, dans lequel le type d'instrument chirurgical correspondant aux données de prédiction d'instrument comportant la situation le résultat reconnu est déduit en utilisant un troisième modèle appris (43A) appris avec un grand nombre de troisièmes données d'enseignement dans lequel les données de prédiction d'instrument sont associées au type d'instrument chirurgical ; et
la déduction d'une position de prise en main qui est une position dans laquelle l'instrument chirurgical prédit est remis au chirurgien,
dans lequel les données de prédiction d'instrument comportent en outre des informations d'identification du chirurgien permettant d'identifier la main dominante du chirurgien et la position de prise en main préférée du chirurgien.
